(19) Europäisches Patentamt
European Patent Office
Office européen des brevets

(11) EP 1 329 512 A1

(12) **EUROPEAN PATENT APPLICATION**
published in accordance with Art. 158(3) EPC

(43) Date of publication:
**23.07.2003 Bulletin 2003/30**

(21) Application number: **01970263.8**

(22) Date of filing: **27.09.2001**

(51) Int Cl.[7]: **C12P 21/06**, C12Q 1/26

(86) International application number:
**PCT/JP01/08483**

(87) International publication number:
**WO 02/027012 (04.04.2002 Gazette 2002/12)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.09.2000 JP 2000296539**

(71) Applicant: **ARKRAY, Inc.**
**Kyoto-shi, Kyoto 601-8045 (JP)**

(72) Inventors:
- **YAGI, Masayuki, c/o ARKRAY, INC.**
  **Kyoto-shi, Kyoto 601-8045 (JP)**
- **ISHIMARU, Kaori, c/o ARKRAY, INC.**
  **Kyoto-shi, Kyoto 601-8045 (JP)**

(74) Representative: **VOSSIUS & PARTNER**
**Siebertstrasse 4**
**81675 München (DE)**

(54) **PROCESS FOR PRODUCING PROTEIN DECOMPOSITION PRODUCT**

(57) A method of producing a protein degradation product is provided, by which a protein (including a peptide) in a sample can be degraded quickly and efficiently. A sample containing a protein is treated with a protease in the presence of the tetrazolium compound to give a protein degradation product. Further, by causing a redox reaction between the glycation site of a glycated protein degradation product obtained by this method and a fructosyl amino acid oxidase, and then determining this redox reaction, it is possible to determine the amount of a glycated protein quickly. As the tetrazolium compound, 2-(4-iodophenyl)-3-(2,4-dinitrophenyl)-5-(2,4-disulfophenyl)-2H-tetrazolium salt etc. can be used.

EP 1 329 512 A1

## Description

### Technical Field

The present invention relates to a method of producing a protein degradation product by treating a protein in a sample with a protease.

### Background Art

Conventionally, for the purpose of detecting a target protein (including a peptide) in a sample or deactivating a function of the protein that affects the determination, a method of degrading the protein with a protease has been employed in various determining methods etc.

For example, attempts now are being made to determine, by an enzymic method, a glycated protein in blood, especially glycated hemoglobin in red blood cells reflecting previous blood glucose levels in vivo, as an important index for the diagnosis, treatment, etc. of diabetes, and the method of degrading a glycated protein with a protease also is employed in such an enzymic method. In the above-mentioned enzymic method, a fructosyl amino acid oxidase (hereinafter, referred to as "FAOD") is reacted with a glycation site of a glycated protein contained in a hemolysate sample so that hydrogen peroxide is generated. The amount of the hydrogen peroxide corresponds to the amount of the glycated protein. After this FAOD treatment, a peroxidase (hereinafter, referred to as "POD") and a reducing agent are added to the sample so that the POD catalyzes a redox reaction between the hydrogen peroxide and the reducing agent. In the case where a color-developing substrate that develops color by oxidation is used as the reducing agent, the amount of the hydrogen peroxide can be determined by measuring the color development, and the amount of the glycated protein in the sample can be known from the amount of the hydrogen peroxide thus determined.

However, FAOD to be reacted with the glycation site acts on a glycated amino acid and a glycated peptide fragment shorter than a glycated protein and a glycated peptide more easily than on the glycated protein and the glycated peptide. Accordingly, with the intention of improving the accuracy of determination, the glycated protein is degraded with a protease in advance so that FAOD can act on the glycation site of the glycated protein more easily.

### Disclosure of Invention

However, a protease has a substrate specificity and the degradation activity thereof differs depending on the substrate to be treated. Accordingly, depending on the type of the target protein, it may take a long time to degrade the target protein so that the determination cannot be carried out quickly. Further, when the target protein is glycated as in the case of the above-mentioned glycated protein, it may be difficult to degrade the glycated protein due to the steric hindrance and the like. For this reason, when determining the glycated protein in the above-mentioned manner, the determination process as a whole takes a long time due to the protease treatment carried out in advance. Therefore, from the viewpoint of applicability in the field of clinical tests etc., there has been a demand for a method by which a glycated protein can be determined quickly

Therefore, it is an object of the present invention to provide a method of producing a protein degradation product, by which a protein in a sample can be degraded quickly and efficiently.

In order to achieve the above object, a method of producing a protein degradation product according to the present invention includes: treating a sample containing a protein with a protease in the presence of a tetrazolium compound. It is to be noted that the term "protein" as used in the present invention includes a peptide.

By treating the sample containing the protein with the protease in the presence of the tetrazolium compound as described above, it becomes possible to degrade the protein in the sample quickly.

The tetrazolium compound is a compound having a tetrazole ring structure. In the producing method of the present invention, tetrazolium compounds as described later can be used as the tetrazolium compound. Preferably, the tetrazolium compound is one containing ring substituents at least at two positions on its tetrazole ring. Most preferably, the tetrazolium compound is 2-(4-iodophenyl)-3-(2,4-dinitrophenyl)-5-(2,4-disulfophenyl)-2H-tetrazolium salt.

In the producing method of the present invention, the amount of the tetrazolium compound added to the sample is not specifically limited and can be decided as appropriate, for example, depending on the type and the added amount of the protease, the type of the sample, the amount of the protein contained in the sample, and the like. More specifically, it is preferable that the tetrazolium compound is added to the sample so that a content of the tetrazolium compound per microliter of the sample is in the range from 0.02 to 10 μmol, more preferably from 0.05 to 2 μmol, and most preferably from 0.1 to 2 μmol, for example.

Further, the ratio of the added amount of the tetrazolium compound to the added amount of the protease is as follows, for example: the tetrazolium compound is added to the sample so that a content of the tetrazolium compound per KU of the protease is in a range from 0.001 to 100 μmol, preferably 0.01 to 20 μmol, and most preferably 0.05 to

5 μmol.

In the producing method of the present invention, it is preferable that the sample is treated with the protease in the presence of the tetrazolium compound and a surfactant. By carrying out the protease treatment under the condition that the surfactant coexists with the tetrazolium compound, the degradation of the protein with the protease can be accelerated still further so that the protein in the sample can be determined quickly.

The surfactant is not specifically limited, and can be, for example, a nonionic surfactant. More specifically, polyoxyethylene alkyl ethers, polyoxyethylene alkylphenyl ethers, sorbitan fatty acid esters, and the like can be used as the surfactant. Among these, polyoxyethylene alkyl ethers and polyoxyethylene alkylphenyl ethers are preferable. Other than these, sodium lauryl sulfate, deoxycholic acid, and the like can be used, for example.

More specifically, polyoxyethylene-p-t-octylphenyl ether such as commercially available Triton-type surfactants and the like; polyoxyethylene sorbitan alkyl ester such as commercially available Tween-type surfactants and the like; and polyoxyethylene alkyl ether such as commercially available Brij-type surfactants and the like can be used, for example. Other than these, polyoxyethylene (10) lauryl ether; polyoxyethylene (9) lauryl ether such as Nikkol BL-9 EX (trade name, available from Wako Pure Chemical Industries, Ltd.: the weight-average degree of polymerization N of polyoxyethylene is 9) and the like; and polyoxyethylene octylphenyl ether such as Tergitol NPX (trade name, available from Nacalai Tesque, Inc.: the weight-average degree of polymerization N of polyoxyethylene is about 10.5), Tergitol NP-40 (trade name, available from Nacalai Tesque, Inc.: the weight-average degree of polymerization N of polyoxyethylene is 20), and the like also can be used, for example.

In the producing method of the present invention, the amount of the tetrazolium compound added to a sample is not specifically limited. For example, the surfactant preferably is added to the sample so that a content of the surfactant per mole of the tetrazolium compound is in the range from 0.01 to 300 mol, more preferably from 0.05 to 150 mol, and most preferably from 0.2 to 100 mol.

On the other hand, the added amount of the surfactant per microliter of the sample preferably is in the range from 0.01 to 50 μmol and more preferably from 0.05 to 10 μmol.

In the producing method of the present invention, it is preferable that the protein to be degraded is a glycated protein. Examples of the glycated protein include glycated hemoglobin, glycated albumin, and the like. Among these, glycated hemoglobin is preferable. Further, if a degradation product of a glycated protein is produced by the producing method of the present invention, the glycated protein can be determined quickly by a method of determining a glycated protein (including a glycated peptide) as described later.

The protease used in the producing method of the present invention is not specifically limited. Examples of the protease include serine proteases, thiol proteases, metalloproteases, and the like. More specifically, it is preferable to use a trypsin, proteinase K, chymotrypsin, papain, bromelain, subtilisin, elastase, aminopeptidase, and the like.

Further, in the case where the protein to be degraded is glycated hemoglobin, a protease that degrades the glycated hemoglobin selectively, e.g., a bromelain, papain, trypsin derived from porcine pancreas, metalloproteinase, and protease derived from *Bacillus subtilis*, preferably is used. Examples of the protease derived from *Bacillus subtilis* include Protease N (trade name, available from Fluka Chemie AG, for example), Protease N "AMANO" (trade name, available from Amano Enzyme Inc.), and the like. Examples of the metalloproteinase include the metalloproteinase (EC 3. 4. 24. 4) derived from the genus *Bacillus* (e.g., available from Toyobo Co., Ltd. under the trade name Toyoteam) and the like. Among these, a metalloproteinase, bromelain, and papain are more preferable, and a metalloproteinase is most preferable. By using the protease that degrades the glycated hemoglobin selectively, a degradation product of a particular glycated protein can be prepared selectively.

In the producing method of the present invention, the amount of the protease added to the sample is not specifically limited and can be decided as appropriate, for example, depending on the type of the protease to be used, the type and the amount of the protein to be degraded, and the like. For example, the protease preferably is added to the sample so that a content of the protease per microliter of the sample is in the range from 10 to 1,000,000 U, more preferably from 100 to 500,000 U, and most preferably from 500 to 200,000 U.

In the producing method of the present invention, the sample is not specifically limited. For example, in addition to blood samples such as whole blood, plasma, serum, blood cells, and the like, biological samples such as urine, spinal fluid, saliva and the like; beverage such as juice and the like; food such as soy sauce, sauce, and the like also can be used as the sample. Among these, blood samples such as whole blood and the like and blood cell samples are preferable.

Next, a method of determining an amount of glycated protein according to the present invention includes: degrading a glycated protein in a sample by the method of producing a protein degradation product according to the present invention to give a glycated protein degradation product; causing a redox reaction between a glycation site of the glycated protein degradation product and FAOD; and determining the redox reaction to determine an amount of the glycated protein. It is to be noted that, as is described above with regard to the term "protein", the term "glycated protein" also includes a glycated peptide.

According to a conventional method in which a glycated protein is treated with a protease in the absence of

a tetrazolium compound, it is necessary to carry out the protease treatment for about 6 to 40 hours in order to degrade the glycated protein sufficiently so as to allow FAOD to act on the glycation site of the glycated protein easily. Accordingly, the above-mentioned enzymic method requires a long time to determine the glycated protein and thus is not very useful. In contrast, according to the method of determining a glycated protein of the present invention, a glycated protein can be determined quickly because it is possible to degrade a protein within a short time. For example, in the case where a glycated protein is determined by the determining method of the present invention under the same conditions as in the conventional method except that a tetrazolium compound is added, the time require for the determination can be reduced to about 1/25 to 1/1200 of that in the case where a tetrazolium compound is not added.

Examples of the tetrazolium compound and the protease used in the determining method of the present invention include those described above. Further, the added amounts of the tetrazolium compound and the protease also are the same as those described above.

In the determining method of the present invention, examples of the glycated protein include those described above. Among these, glycated hemoglobin is most preferable. Hemoglobin is contained in blood at a high concentration of about 60 to 200 g/L, and besides, it is difficult to degrade hemoglobin. Therefore, conventionally, a protease treatment of glycated hemoglobin takes several hours to several days. In contrast, according to the determining method of the present invention, the protease treatment can be carried out within 20 seconds to 2 hours, which allows the glycated hemoglobin to be determined quickly.

In the determining method of the present invention, it is preferable that determining the redox reaction is determining an amount of hydrogen peroxide generated by the redox reaction between the glycation site of the glycated protein degradation product and the FAOD.

It is preferable that the amount of the hydrogen peroxide is determined using an oxidase and a substrate that develops color (i.e., a color-developing substrate) by oxidation. The oxidase is not specifically limited. However, POD preferably is used as the oxidase. Also, the color-developing substrate, is not specifically limited. However, N-(carboxymethylaminocarbonyl)-4,4'-bis(dimethylamino) diphenylamine sodium (for example, the trade name DA-64: available from Wako Pure Chemical Industries, Ltd.), for example, preferably is used as the color-developing substrate because it can be detected with high sensitivity.

The sample to be used in the determining method of the present invention is not specifically limited. Examples of the sample to be used in this method include those described above.

In the determining method of the present invention, FAOD catalyzing a reaction represented by Formula (1) below preferably is used.

$$R^1\text{-CO-CH}_2\text{-NH-}R^2 + H_2O + O_2$$

$$\rightarrow R^1\text{-CO-CHO} + NH_2 - R^2 + H_2O_2 \qquad (1)$$

In Formula (1), $R^1$ denotes a hydroxyl group or a residue derived from a sugar that is not yet subjected to the glycation reaction (i.e., sugar moiety). The sugar moiety ($R^1$) is an aldose residue when the unreacted sugar is aldose, and is a ketose residue when the unreacted sugar is ketose. When the unreacted sugar is glucose, for example, the sugar in the glycated product takes on the fructose structure after the glycation reaction due to Amadori rearrangement. In this case, the sugar moiety ($R^1$) is a glucose residue (aldose residue). This sugar moiety ($R^1$) can be represented, for example, by

$$\text{-[CH(OH)]}_n\text{-CH}_2\text{OH}$$

where n denotes an integer of 0 to 6.

In Formula (1), $R^2$ is not specifically limited. However, in the case where the substrate is a glycated amino acid, a glycated peptide, or a glycated protein, for example, $R^2$ varies depending on which of an $\alpha$-amino group and an amino group other than the $\alpha$-amino group is glycated.

In Formula (1), in the case where an $\alpha$-amino group is glycated, $R^2$ is an amino acid residue or a peptide residue represented by Formula (2) below.

$$\text{-CHR}^3\text{-CO-R}^4 \qquad (2)$$

In Formula (2), $R^3$ denotes an amino-acid side chain group. $R^4$ denotes a hydroxyl group, an amino acid

residue, or a peptide residue, and can be represented, for example, by Formula (3) below. In Formula (3), n denotes an integer of 0 or more and $R^3$ denotes an amino-acid side chain group as described above.

$$-(NH-CHR^3-CO)_n-OH \qquad (3)$$

In Formula (1), in the case where an amino group other than the α-amino group is glycated (i.e., an amino-acid side chain group is glycated), $R^2$ is represented by Formula (4) below.

$$-R^5-CH(NH-R^6)-CO-R^7 \qquad (4)$$

In Formula (4), $R^5$ denotes a portion other than the glycated amino group in the amino-acid side chain group. For example, in the case where the glycated amino acid is lysine, $R^5$ is as follows.

$$-CH_2-CH_2-CH_2-CH_2-$$

On the other hand, in the case where the glycated amino acid is arginine, for example, $R^5$ is as follows.

$$-CH_2-CH_2-CH_2-NH-CH(NH_2)-$$

In Formula (4), $R^6$ denotes hydrogen, an amino acid residue, or a peptide residue, and can be represented, for example, by Formula (5) below. In Formula (5), n denotes an integer of 0 or more and $R^3$ denotes an amino-acid side chain group as described above.

$$-(CO-CHR^3-NH)_n-H \qquad (5)$$

In Formula (4), $R^7$ denotes a hydroxyl group, an amino acid residue, or a peptide residue, and can be represented, for example, by Formula (6) below. In Formula (6), n denotes an integer of 0 or more and $R^3$ denotes an amino-acid side chain group as described above.

$$-(NH-CHR^3-CO)_n-OH \qquad (6)$$

## Best Mode for Carrying Out the Invention

The tetrazolium compound to be used in a method of producing a protein degradation product according to the present invention preferably contains ring substituents at least at two positions on its tetrazole ring, more preferably at three positions on its tetrazole ring, for example.

In the case where the tetrazolium compound contains ring substituents at least at two positions on its tetrazole ring as described above, it is preferable that the ring substituents are at the 2-position and 3-position on the tetrazole ring. Further, in the case where the tetrazolium compound contains ring substituents at three positions on its tetrazole ring, it is preferable that the ring substituents are at the 2-position, 3-position, and 5-position on the tetrazole ring.

Further, it is preferable that at least two ring substituents of the tetrazolium compound have a benzene ring structure. Other than the benzene ring structure, the ring substituents may have a resonance structure with S or O being contained in the ring skeleton, for example. Examples of the ring substituents with such a resonance structure include a thienyl group, thiazoyl group, and the like.

Furthermore, it is preferable that the tetrazolium compound contains ring substituents at least at three positions on its tetrazole ring and at least two of the ring substituents have a benzene ring structure.

Still further, it is preferable that at least one ring substituent of the tetrazolium compound contains a functional group, and a larger number of functional groups are more preferable.

As the functional group, an electron-withdrawing functional group preferably is used. For example, a halogen group, ether group, ester group, carboxy group, acyl group, nitroso group, nitro group, hydroxy group, sulfo group, and the like can be used. Other than these, characteristic groups containing oxygen such as a hydroperoxy group, oxy

group, epoxy group, epidioxy group, oxo group, and the like; and characteristic groups containing sulfur such as a mercapto group, alkylthio group, methylthiomethyl group, thioxo group, sulfino group, benzenesulfonyl group, phenylsulfonyl group, p-toluenesulfonyl group, p-tolylsulfonyl group, tosyl group, sulfamoyl group, isothiocyanate group, and the like also can be used, for example. Among these electron-withdrawing functional groups, a nitro group, sulfo group, halogen group, carboxy group, hydroxy group, methoxy group, and ethoxy group are preferable. Further, in addition to the above-mentioned electron-withdrawing functional groups, unsaturated hydrocarbon groups such as a phenyl group ($C_6H_5$-), styryl group ($C_6H_5CH = CH$-), and the like also can be used, for example. It is to be noted that the functional groups may have been ionized by dissociation.

Still further, it is preferable that the tetrazolium compound contains benzene rings at the 2-position and 3-position on its tetrazole ring and at least one of the benzene rings contains at least one functional group selected from the group consisting of a halogen group, carboxy group, nitro group, hydroxy group, sulfo group, methoxy group, and ethoxy group. It is to be noted here that both the benzene rings may contain the functional group. Further, the functional group may be contained at any positions (ortho-, meta-, para-) on the benzene ring. Furthermore, the number of the functional group is not specifically limited, and in the case where the at least one of the benzene rings contains a plurality of functional groups, the functional groups contained in the benzene ring may be the same or different.

Examples of the tetrazolium compound containing ring substituents having a benzene ring structure at the 2-position, 3-position, and 5-position on its tetrazole ring include:

2-(4-iodophenyl)-3-(4-nitrophenyl)-5-(2,4-disulfophenyl)-2H-tetrazolium salt;
2-(4-iodophenyl)-3-(2,4-dinitrophenyl)-5-(2,4-disulfophenyl)-2H-tetrazolium salt;
2-(2-methoxy-4-nitrophenyl)-3-(4-nitrophenyl)-5-(2,4-disulfophenyl)-2H-tetrazolium salt;
2-(4-iodophenyl)-3-(4-nitrophenyl)-5-phenyl-2H-tetrazolium salt;
3,3'-(1,1'-biphenyl-4,4'-diyl)-bis(2,5-diphenyl)-2H-tetrazolium salt;
3,3'-[3,3'-dimethoxy-(1,1'-biphenyl)-4,4'-diyl] -bis[2-(4-nitrophenyl)-5-phenyl-2 H-tetrazolium salt];
2,3-diphenyl-5-(4-chlorophenyl) tetrazolium salt;
2,5-diphenyl-3-(p-diphenyl) tetrazolium salt;
2,3-diphenyl-5-(p-diphenyl) tetrazolium salt;
2,5-diphenyl-3-(4-styrylphenyl) tetrazolium salt;
2,5-diphenyl-3-(m-tolyl) tetrazolium salt; and
2,5-diphenyl-3-(p-tolyl) tetrazolium salt.

The tetrazolium compound is not limited to those described above. In addition to the above-mentioned tetrazolium compounds, a tetrazolium compound containing ring substituents having a benzene ring structure at two positions and one ring substituent having a structure other than the benzene ring structure at one position on its tetrazole ring also may be used. Examples of such a tetrazolium compound include:

2,3-diphenyl-5-(2-thienyl) tetrazolium salt;
2-benzothiazoyl-3- (4-carboxy-2-methoxyphenyl)-5-[4-(2-sulfoethyl carbamoyl) phenyl]-2H-tetrazolium salt;
2,2'-dibenzothiazoyl-5,5'-bis [4-di(2-sulfoethyl) carbamoylphenyl]-3,3'-(3,3'-dimethoxy-4,4'-biphenylene) ditetrazolium salt; and
3-(4,5-dimethyl-2-thiazoyl)-2,5-diphenyl-2H-tetrazolium salt.

Further, a tetrazolium compound containing ring substituents having a benzene ring structure at two positions and one substituent not having a ring structure at one position on its tetrazole ring also can be used.
Examples of such a tetrazolium compound include:

2,3-diphenyl-5-cyano tetrazolium salt;
2,3-diphenyl-5-carboxy tetrazolium salt;
2,3-diphenyl-5-methyltetrazolium salt; and
2,3-diphenyl-5-ethyl tetrazolium salt.

Among the above-mentioned tetrazolium compounds, the tetrazolium compounds containing three ring substituents are preferable as described above. Among these, the tetrazolium compounds containing three ring substituents having a benzene ring structure and a large number of electron-withdrawing functional groups is more preferable, and 2-(4-iodophenyl)-3-(2,4-dinitrophenyl)-5-(2,4-disulfophenyl)-2H-tetrazolium salt is most preferable. It is to be noted here that the above-mentioned tetrazolium compounds may be a salt or may have been ionized, for example. Further, the tetrazolium compounds may be used alone or in combinations of two or more types.

Hereinafter, a method of producing a protein degradation product according to the present invention will be

described by taking as an example the case where a glycated protein in blood cells is degraded.

First, a hemolysate sample is prepared by causing hemolysis of whole blood or of a blood cell fraction separated from the whole blood according to a usual method such as centrifugation and the like. The method of causing hemolysis is not specifically limited, and can be, for example, a method using a surfactant, a method using ultrasonic waves, and a method utilizing the difference in osmotic pressure. Among these, the method using a surfactant is preferable.

The surfactant to be used for causing hemolysis is not specifically limited. For example, as the surfactant, nonionic surfactants such as polyoxyethylene-p-t-octylphenyl ether (Triton-type surfactant etc.), polyoxyethylene sorbitan alkyl ester (Tween-type surfactant etc.), polyoxyethylene alkyl ether (Brij-type surfactant etc.), and the like can be used. More specifically, Triton X-100 (trade name), Tween-20 (trade name), Brij 35 (trade name), and the like can be used, for example. Generally, the treatment with the surfactant can be carried out under the following conditions: in the case where the solution to be treated contains 1 to 10 vol% of blood cells, the surfactant is added to the solution so as to give a concentration of 0.1 to 1 wt% and the resultant mixture is stirred at room temperature for about 5 seconds to 1 minute.

Further, when utilizing the difference in osmotic pressure, to the whole blood is added 2 to 100 times its volume of purified water to cause hemolysis, for example.

Subsequently, the hemolysate sample is pretreated with the protease in the presence of the tetrazolium compound. As a result, a glycated protein degradation product is obtained.

Generally, this pretreatment with the protease is carried out in a buffer. Further, the type of the buffer is not specifically limited, and can be, for example, Tris-HCl buffer, EPPS buffer, PIPES buffer, phosphate buffer, ADA buffer, citrate buffer, acetate buffer, glycineamide buffer, CHES buffer, and the like. The pH of the buffer preferably is in the range from 5 to 12, more preferably from 6 to 10, and most preferably from 7 to 9.

The protease is added to the sample at the following ratio, for example: in the case where the solution to be pretreated contains 0.1 to 10 vol% of blood cells, the protease preferably is added to the solution so as to give a concentration in the range from 0.1 to 100 g/L, more preferably from 0.3 to 50 g/L, and most preferably from 0.5 to 20 g/L.

Further, in the case where the glycated protein to be degraded is glycated hemoglobin, a protease that degrades the glycated hemoglobin selectively, for example, is used as the protease. As the protease that degrades the glycated hemoglobin selectively, a bromelain, papain, trypsin derived from porcine pancreas, metalloproteinase, and protease derived from *Bacillus subtilis* preferably are used. The protease that degrades the glycated hemoglobin selectively is added to the sample at the following ratio, for example: in the case where the solution to be pretreated contains 0.1 to 10 vol% of blood cells, the protease preferably is added to the solution so as to give a concentration in the range from 0.1 to 50 g/L, more preferably from 0.3 to 30 g/L, and most preferably from 1 to 20 g/L. More specifically, in the case where a metalloproteinase is used as the protease, the metalloproteinase preferably is added to the solution to be pretreated containing 0.3 to 5 vol% of blood cells so as to give a concentration in the range from 0.1 to 30 g/L, more preferably from 0.3 to 20 g/L, and most preferably from 1 to 10 g/L.

The tetrazolium compound is added to the sample at the following ratio, for example: in the case where the solution to be pretreated contains 0.1 to 10 vol% of blood cells, the tetrazolium compound preferably is added to the solution so as to give a concentration in the range from 0.1 to 50 mmol/L, more preferably from 0.2 to 20 mmol/L, and most preferably from 0.3 to 10 mmol/L. More specifically, in the case where the tetrazolium compound is 2-(4-iodophenyl)-3-(2,4-dinitrophenyl)-5- (2,4-disulfophenyl)-2H-tetrazolium salt and the solution to be pretreated contains 0.1 to 10 vol% of blood cells, the tetrazolium compound preferably is added to the solution so as to give a concentration in the range from 0.3 to 15 mmol/L, more preferably from 0.5 to 15 mmol/L, and most preferably from 1 to 10 mmol/L.

The tetrazolium compound may be used as it is. However, on account of the ease of operation, treatment efficiency, etc., it is preferable to use a tetrazolium compound solution obtained by dissolving the tetrazolium compound in a solvent. The concentration of the tetrazolium compound in the solution can be decided as appropriate, for example, depending on the type of the tetrazolium compound to be used (e.g., the molecular weight thereof) and the like. For example, the concentration of the tetrazolium compound in the solution is in the range from 0.01 to 120 mmol/L, preferably from 0.1 to 50 mmol/L, and more preferably from 0.2 to 20 mmol/L. As the solvent, distilled water, a physiological salt solution, buffers, and the like can be used, for example. Examples of the buffers include those described above. Further, the tetrazolium compound may be used alone or in combinations of two or more types.

The conditions of the protease treatment carried out in the presence of the tetrazolium compound are not specifically limited. For example, the protease treatment is carried out at a temperature in the range from 10°C to 37°C and the treatment time in the range from 30 seconds to 60 minutes; preferably at a temperature in the range from 20°C to 37°C and the treatment time in the range from 30 seconds to 10 minutes; and more preferably at a temperature in the range from 25°C to 37°C and the treatment time in the range from 30 seconds to 5 minutes.

By treating a sample with the protease in the presence of the tetrazolium compound as describe above, degradation of a glycated protein in the sample can be accelerated. Accordingly, a degradation product of the glycated protein can be obtained within a short time and besides, with high degradation efficiency.

The protease treatment may be carried out not only in the presence of the tetrazolium compound as described above but also in the presence of both the tetrazolium compound and a surfactant. When the protease treatment is carried out under the condition that the surfactant coexists with the tetrazolium compound, the degradation of a glycated protein in the sample can be further accelerated.

The amount of the surfactant added to the sample is not specifically limited. For example, the surfactant may be added to the sample at the following ratio: in the case where the solution to be pretreated contains 0.3 to 5 vol% of blood cells, the surfactant preferably is added to the solution so as to give a concentration in the range from 0.1 to 500 mmol/L, more preferably from 0.5 to 150 mmol/L, and most preferably from 1 to 100 mmol/L. More specifically, in the case where the surfactant is Triton X-100 (trade name), the surfactant preferably is added to the solution so as to give a concentration in the range from 0.2 to 400 mmol/L, more preferably from 1 to 150 mmol/L, and most preferably from 2 to 100 mmol/L. On the other hand, in the case where the surfactant is Brij 35 (trade name), the surfactant preferably is added to the solution so as to give a concentration in the range from 0.1 to 200 mmol/L, more preferably from 0.5 to 10 mmol/L, and most preferably from 1 to 50 mmol/L.

The surfactant may be added to the sample so that a content of the surfactant per millimole of the tetrazolium compound is in the range from 0.01 to 300 mmol, more preferably from 0.05 to 150 mmol, and most preferably from 0.2 to 100 mmol, for example.

More specifically, in the case where the surfactant is Triton X-100 (trade name), the surfactant is added to the sample so that a content of the surfactant per 1.5 mmol of the tetrazolium compound is in the range from 0.2 to 400 mmol, more preferably from 1 to 150 mmol, and most preferably from 2 to 100 mmol. On the other hand, in the case where the surfactant is Brij 35 (trade name), the surfactant is added to the sample so that a content of the surfactant per 1.5 mmol of the tetrazolium compound is in the range from 0.1 to 200 mmol, more preferably from 0.5 to 150 mmol, and most preferably from 1 to 50 mmol.

Further, in the case where hemolysis of the sample is caused by treating the sample with the surfactant as described above, the surfactant may previously be added to the sample during this hemolysis treatment so as to give a sufficient surfactant concentration for the pretreatment with the protease.

Hereinafter, a method of determining a glycated protein according to the present invention will be described by taking as an example the case where the amount of a glycated protein in blood cells is determined.

First, a hemolysate sample is prepared from a whole blood sample or a blood cell sample in the same manner as described above. Then, the hemolysate sample is treated with a protease in the presence of a tetrazolium compound in the same manner as described above to prepare a degradation product of a glycated protein. As described above, FAOD acts on a glycated amino acid and a glycated peptide fragment shorter than the glycated protein more easily than on the glycated protein itself. On this account, the protease treatment is carried out to degrade the glycated protein so that FAOD can act on a glycation site of the glycated protein more easily. By treating the sample with the protease in the presence of the tetrazolium compound as described above, it becomes possible to degrade the glycated protein within a short time and besides, with high degradation efficiency. Accordingly, FAOD can act on the glycation site sufficiently even though the protease treatment is carried out only for a short time. The protease treatment may be carried out in the presence of a tetrazolium compound and a surfactant because this allows degradation of the glycated protein to be further accelerated.

Next, the degradation product obtained through the above-mentioned protease treatment is treated with the FAOD. This FAOD treatment catalyzes the reaction represented by Formula (1) above.

Similarly to the above-mentioned protease treatment, this FAOD treatment preferably is carried out in a buffer. The conditions of the FAOD treatment are decided as appropriate depending on the type of the FAOD to be used, the type and the concentration of the glycated protein to be determined, and the like.

More specifically, the FAOD treatment is carried out under the following conditions, for example: the FAOD concentration in the reaction solution in the range from 50 to 50,000 U/L, the blood cell concentration in the reaction solution in the range from 0.01 to 1 vol%, the reaction temperature in the range from 15°C to 37°C, the reaction time in the range from 1 to 60 minutes, and the pH in the range from 6 to 9. The type of the buffer is not specifically limited, and the same buffers as used in the protease treatment also can be used in the FAOD treatment.

Next, using POD and the color-developing substrate, the amount of the hydrogen peroxide generated by the FAOD treatment is determined utilizing a redox reaction.

As the color-developing substrate, N-(carboxymethylaminocarbonyl)-4,4'-bis(dimethylamino)diphenylamine sodium, orthophenylenediamine (OPD), a substrate obtained by combining a Trinder's reagent and 4-aminoantipyrine, and the like can be used, for example. Examples of the Trinder's reagent include phenols, phenol derivatives, aniline derivatives, naphthols, naphthol derivatives, naphthylamine, naphthylamine derivatives, and the like. Further, in place of the above-mentioned 4-aminoantipyrine, it is possible to use aminoantipyrine derivatives, vanillin diamine sulfonic acid, methyl benzothiazolinone hydrazone (MBTH), sulfonated methyl benzothiazolinone hydrazone (SMBTH), and the like. Among these color-developing substrates, N-(carboxymethylaminocarbonyl)-4,4'-bis (dimethylamino)diphenylamine sodium is most preferable as described above.

The above-mentioned redox reaction generally is induced in a buffer under the conditions decided as appropriate depending on the concentration of the hydrogen peroxide generated and the like. Generally, the redox reaction is induced under the following conditions: the concentration of POD in the reaction solution in the range from 10 to 20,000 IU/L, the concentration of the color-developing substrate in the range from 0.001 to 1 mmol/L, the reaction temperature in the range from 20°C to 37°C, the reaction time in the range from 1 to 5 minutes, and the pH in the range from 6 to 9. Further, the buffer is not specifically limited, and the same buffers as used in the protease treatment, the FAOD treatment, etc. also can be used.
For example, in the case where the color-developing substrate is used in the redox reaction, the amount of the hydrogen peroxide can be determined by measuring the degree of color development (i.e., the absorbance) of the reaction solution with a spectrophotometer. The amount of the glycated protein in the sample can be determined using the concentration of the hydrogen peroxide thus determined and a calibration curve etc.
It is to be noted here that the amount of the hydrogen peroxide can be determined not only by the above-mentioned enzymic method using the POD etc. but also by an electrical method, for example.
The method of determining a glycated protein according to the present invention enables quick determination as described above. Further, according to a conventional method, a shortened treatment time of the protease treatment may cause the accuracy of determination to be degraded. In contrast, according to the method of the present invention, the glycated protein can be determined with high accuracy within a short time.

EXAMPLES

Hereinafter, examples of the present invention will be described along with comparative examples.

Example 1 and Comparative Example 1

In Example 1, hemoglobin (Hb) was treated with a protease in the presence of a tetrazolium compound, and the amount of a glycation product contained in the thus-obtained hemoglobin degradation product was determined to examine the degree of Hb degradation. The samples, reagents, and method used in the present example will be described in the following.

(Preparation of Hb)

Whole blood was collected from a healthy subject and washed with a 0.9 wt% NaCl aqueous solution 3 to 5 times. Then, the blood cells were diluted 5-fold (by volume) with distilled water to cause hemolysis. The hemolysate was then centrifuged (7000 G, 30 min) and the supernatant (about 75 to 200 mL) was collected as a hemolysate sample. About 30 g of a cation exchange gel (the trade name POROS HS/50: available from Applied Biosystems) was swelled in 500 mL of a 20 mM sodium phosphate buffer (pH 6.0). Then, the supernatant was added to the cation exchange gel and the resultant mixture was stirred so that Hb is adsorbed onto the gel. The mixture was allowed to stand for about 60 minutes and thereafter, the mixture was filtered through a funnel having a diameter of 95 mm and the gel was collected. Then, the collected gel was washed with the sodium phosphate buffer until a filtrate thereof became transparent. Subsequently, Hb was eluted with a 50 mM sodium phosphate buffer (pH 7.5) and the eluted fraction was collected (about 300 to 400 ml). The eluted fraction thus obtained was used as a purified Hb sample. The Hb concentration of the sample was determined by the usual method (e.g., SLS method and the like). The storage temperature of the sample was -80°C.

(Protease solutions)

Proteinase K (trade name, available from Wako Pure Chemical Industries, Ltd.), Toyoteam (trade name, available from Toyobo Co., Ltd.), Protease N "AMANO" (trade name, available from Amano Enzyme Inc.), and Papain (trade name, available from Hoffmann-La Roche Inc.) were dissolved in purified water, respectively, to prepare protease solutions (2g/L).

(WST-3 solution: hereinafter the same)

2-(4-iodophenyl)-3-(2,4-dinitrophenyl)-5-(2,4-disulfophenyl)-2H-tetrazolium monosodium salt (the trade name WST-3: available from Dojindo Laboratories) represented by Formula (7) below was dissolved in purified water so as to give a concentration of 5 mM to prepare a WST-3 solution.

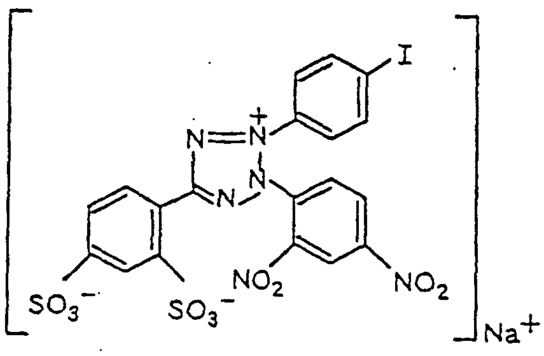

WST-3

(7)

(Buffer)

80 mM glycine-sodium buffer (pH 10, pH 11)

(Composition of Protease Reaction Solution)

| | Example 1 | Comparative Example 1 | Final Concentration |
|---|---|---|---|
| Purified Hb sample | 50 µl | 50 µl | 1.87 mg/ml |
| Purified water | 250 µl | 350 µl | - |
| Buffer | 50 µl | 50 µl | 8.0 mM |
| Protease solution | 50 µl | 50 µl | 0.2 g/L |
| WST-3 solution | 100 µl | - | 1.7 mM |
| Total volume | 500 µl | 500 µl | |

(Reagent A)

| | |
|---|---|
| POD (Toyobo Co., Ltd., hereinafter the same) | 20 KU/L |
| DA 64 (trade name, Wako Pure Chemical Industries, Ltd.) | 40 µmol/L |
| Potassium phosphate buffer (KPB) pH 7.0 | 0.1 mol/L |

(Reagent B)

| | |
|---|---|
| FAOD (Asahi Chemical Industry Co., Ltd.) | 14.6 KU/L |
| KPB | 0.1 mol/L |

(Method)

Protease reaction solutions having the above-mentioned composition were prepared using the above-mentioned respective proteases. The protease reaction solutions were reacted at 37°C for a predetermined time (0 min, 10 min, 30 min, and 60 min). Thereafter, the reaction solutions were cooled with ice to stop the reaction. The reaction solutions were supplied to an ultrafiltration membrane (10,000 molecular weight cut off, 7000 G, 60 min, 4°C) and filtrates were collected.
Subsequently, 45 µl of the reagent A was added to 25 µl of the respective filtrates, and 20 µl of the reagent B was further added after 5 minutes. Then, assuming the time point at which the reagent B was added to be 0 minute,

an increase in absorbance of these filtrates after 3 minutes was measured using a biochemical automatic analysis apparatus (the trade name JCA-BM 8: available from Japan Electron Optics Laboratory Co. Ltd.). The absorbance was measured using, as a measurement wavelength, a main wavelength of 751 nm and a sub-wavelength of 884 nm. Table 1 below shows the absorbance of the filtrates according to Example 1 in which the protease treatment was carried out in the presence of WST-3. It is to be noted here that the absorbance measured 0 minute after the start of the reaction was assumed to be 0.000. As Comparative Example 1, the absorbance of the filtrates was measured in the same manner as in Example 1 except that purified water was added in place of WST-3 as shown in the above-mentioned "Composition of Protease Reaction Solution".

(Table 1)

| Protease | pH of reaction solution | Time for protease treatment 10 min | 30 min | 60 min |
|---|---|---|---|---|
| Proteinase K | 10 | 0.22 | - | - |
| | 11 | 0.011 | 0.018 | - |
| Toyoteam | 10 | 0.004 | 0.007 | 0.010 |
| | 11 | 0.002 | 0.004 | 0.004 |
| Protease N "AMANO" | 10 | 0.003 | 0.011 | 0.017 |
| | 11 | 0.004 | 0.004 | 0.005 |
| Papain | 10 | 0.000 | 0.002 | 0.004 |

In Comparative Example 1, no increase in absorbance (absorbance: 0.00) was observed even when the protease treatment was carried out for 60 minutes, and only a slight increase in absorbance (absorbance: 0.000 to 0.004) was observed when the protease treatment was carried out for 3 to 6 hours. In contrast, in Example 1 in which the protease treatment was carried out in the presence of WST-3, an increase in absorbance with time as show in Table 1 was observed because Hb was degraded by the protease treatment carried out for 10 to 60 minutes.

<u>Example 2</u>

In Example 2, hemoglobin was treated with a protease in the presence of WST-3 and a surfactant, and the degree of degradation of Hb was examined. The samples, reagents, and method used in the present example will be described in the following. Unless otherwise stated, the process for examining the degree of degradation of Hb was the same manner as that in Example 1.

(Surfactant Solutions)

Triton X-100 (trade name, available from Wako Pure Chemical Industries) and Brij 35 (trade name, available from Sigma Chemical Co.) were added to purified water, respectively, so as to give a concentration of 0.3 wt% to prepare surfactant solutions.

The protease reaction was induced in the same manner as in Example 1 except that 250 μl of the above-mentioned surfactant solutions were used in place of 250 μl of purified water (the final concentration of the surfactants: 0.15 wt%), and the absorbance was measured also in the same manner as in Example 1. Table 2 below shows the results in the case where hemoglobin was treated in the presence of WST-3 and Triton X-100, and Table 3 below shows the results in the case where hemoglobin was treated in the presence of WST-3 and Brij 35.

(Table 2)

| WST-3 and Triton X-100 | | | | |
|---|---|---|---|---|
| Protease | pH of reaction solution | Time for protease treatment | | |
| | | 10 min | 30 min | 60 min |
| Proteinase K | 11 | 0.020 | 0.021 | 0.019 |
| Toyoteam | 10 | 0.014 | 0.018 | 0.020 |
| Protease N "AMANO" | 10 | 0.021 | 0.022 | 0.022 |
| Papain | 10 | 0.003 | 0.005 | 0.005 |

(Table 3)

| WST-3 and Brij 35 | | | | |
|---|---|---|---|---|
| Protease | pH of reaction solution | Time for protease treatment | | |
| | | 10 min | 30 min | 60 min |
| Proteinase K | 11 | 0.023 | 0.021 | 0.021 |
| Toyoteam | 10 | 0.012 | 0.015 | 0.016 |
| Protease N "AMANO" | 10 | 0.018 | 0.018 | 0.020 |

As can be seen from Tables 2 and 3, in the case where the surfactant was used in combination with WST-3 when carrying out the protease treatment in the presence of WST-3, the increase in absorbance was even greater than that observed in Example 1. Further, after the absorbance reached around 0.020, no further increase in absorbance was observed. The reason for this is considered that the reaction was terminated.

As described above, when a protease treatment is carried out in the presence of WST-3, degradation of a protein is accelerated so that the protein can be degraded within a short time and with high degradation efficiency. Further, by using a surfactant in combination with WST-3, the protease treatment can be accelerated still further.

Industrial Applicability

As specifically described above, a method of producing a protein degradation product according to the present invention can degrade a protein in a sample quickly by treating the sample with a protease in the presence of a tetrazolium compound. Therefore, according to a method of determining a glycated protein of the present invention employing the method of producing a protein degradation product, determination of glycated hemoglobin in red blood cell, which is important for the diagnosis of diabetes, can be carried out quickly.

## Claims

**1.** A method of producing a protein degradation product comprising:

treating a sample containing a protein with a protease in the presence of a tetrazolium compound.

**2.** The method according to claim 1,
wherein the tetrazolium compound contains benzene rings at a 2-position and 3-position on its tetrazole ring, and at least one of the benzene rings contains at least one functional group selected from the group consisting of a halogen group, a carboxy group, a nitro group, a hydroxy group, a sulfo group, a methoxy group, and an ethoxy group.

**3.** The method according to claim 1,
wherein the tetrazolium compound is 2-(4-iodophenyl)-3-(2,4-dinitrophenyl)-5-(2,4-disulfophenyl)-2H-tetrazolium salt.

**4.** The method according to claim 1,
wherein the tetrazolium compound is added to the sample so that a content of the tetrazolium compound per microliter of the sample is in a range from 0.02 to 10 μmol.

**5.** The method according to claim 1,
wherein the protease is added to the sample so that a content of the protease per microliter of the sample is in a range from 10 to 1,000,000 U.

**6.** The method according to claim 1,
wherein the tetrazolium compound is added to the sample so that a content of the tetrazolium compound per KU of the protease is in a range from 0.001 to 100 μmol.

**7.** The method according to claim 1,

wherein the sample is treated with the protease in the presence of the tetrazolium compound and a surfactant.

**8.** The method according to claim 7,
wherein the surfactant is a nonionic surfactant.

**9.** The method according to claim 8,
wherein the nonionic surfactant is at least one surfactant selected from the group consisting of polyoxyethylene alkyl ethers, polyoxyethylene alkylphenyl ethers, and sorbitan fatty acid esters.

**10.** The method according to claim 7,
wherein the surfactant is added to the sample so that a content of the surfactant per millimole of the tetrazolium compound is in the range from 0.01 to 300 mmol.

**11.** The method according to claim 7,
wherein the surfactant is added to the sample so that a content of the surfactant per microliter of the sample is in a range from 0.01 to 50 μmol.

**12.** The method according to claim 1,
wherein the protease is at least one protease selected from the group consisting of trypsins, proteinase K, chymotrypsins, papains, bromelains, subtilisins, elastases, and aminopeptidases.

**13.** The method according to claim 1,
wherein the protease is a protease that degrades glycated hemoglobin selectively, the protease that degrades glycated hemoglobin selectively being at least one protease selected from the group consisting of bromelains, papains, trypsins derived from porcine pancreas, metalloproteinases, and proteases derived from *Bacillus subtilis*.

**14.** The method according to claim 1,
wherein the protein to be degraded is a glycated protein.

**15.** The method according to claim 14,
wherein the glycated protein is glycated hemoglobin.

**16.** The method according to claim 1,
wherein the sample is whole blood.

**17.** The method according to claim 1,
wherein the sample contains blood cells.

**18.** A method of determining an amount of glycated protein comprising:

degrading a glycated protein in a sample by the method of producing a protein degradation product according to claim 1 to give a glycated protein degradation product;
causing a redox reaction between a glycation site of the glycated protein degradation product and a fructosyl amino acid oxidase; and
determining the redox reaction to determine an amount of the glycated protein.

**19.** The method according to claim 18,
wherein determining the redox reaction is determining an amount of hydrogen peroxide generated by the redox reaction between the glycation site of the glycated protein degradation product and the fructosyl amino acid oxidase.

**20.** The method according to claim 19,
wherein the amount of the hydrogen peroxide is determined using the hydrogen peroxide and a substrate that develops color by oxidation.

**21.** The method according to claim 20,
wherein determining the amount of the hydrogen peroxide is measuring an absorbance of the substrate that has developed color as a result of a reaction caused by the oxidase between the hydrogen peroxide and the substrate.

## INTERNATIONAL SEARCH REPORT

International application No.
PCT/JP01/08483

A. CLASSIFICATION OF SUBJECT MATTER
Int.Cl[7] C12P21/06, C12Q1/26

According to International Patent Classification (IPC) or to both national classification and IPC

B. FIELDS SEARCHED

Minimum documentation searched (classification system followed by classification symbols)
Int.Cl[7] C12P21/06, C12Q1/26

Documentation searched other than minimum documentation to the extent that such documents are included in the fields searched

Electronic data base consulted during the international search (name of data base and, where practicable, search terms used)
WPI(DIALOG),BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| X | EP 1002874 A2 (KDK CORPORATION), 24 May, 2000 (24.05.00), & JP 2000-210100 A | 1-21 |
| A | JP 11-196896 A (Dojin Kagaku Kenkyusho K.K.), 27 July, 1999 (27.07.99) (Family: none) | 1-21 |
| A | EP 848066 A1 (Merck Patent Gesellschaft), 17 June, 1998 (17.06.98), & JP 10-179194 A & US 6004769 A | 1-21 |
| A | EP 574056 A1 (Eastman Kodak Company), 15 December, 1993 (15.12.93), & JP 6-46896 A & US 5981206 A | 1-21 |
| A | JP 61-54455 A (Konishiroku Photo Ind. Co., Ltd.), 18 March, 1986 (18.03.86) (Family: none) | 1-21 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 December, 2001 (18.12.01) | 25 December, 2001 (25.12.01) |
| Name and mailing address of the ISA/ Japanese Patent Office | Authorized officer |
| Facsimile No. | Telephone No. |

Form PCT/ISA/210 (second sheet) (July 1992)